# EUROPEAN PATENT APPLICATION

(11) **EP 2 551 357 A1**
(43) Date of publication of application: **30.01.2013**
(21) Application number: 10848182.1
(22) Date of filing: 24.03.2010
(51) Int. Cl.: C13K 1/02, B01J 23/40, B01J 31/02, B01J 31/00, C08B 15/08, C08B 1/00

(54) **SIMULTANEOUS HYDROLYSIS REFINE METHOD OF CELLULOSE BIOMASS**

(71) Applicant: China Fuel (Huaibei) Bioenergy Technology Development Co., Ltd, Anhui 235000 (CN); Sun Pharmaceuticals, Inc., San Diego, CA 92128 (US)
(72) Inventor: ZHU, Zuolin, San Diego, California 92128 (US); SUN, Meg M., San Diego, California 92128 (US); SU, Chungao, Huaibei Anhui 235000 (CN); YE, Hongping, Huaibei Anhui 235000 (CN)
(74) Representative: Biggi, Cristina
(86) International application number: PCT/CN2010/071254
(87) International publication number: WO 2011/116520

(57) **Abstract**

A method of refining cellulosic biomass, comprising synchronous hydrolyzation of at least 85wt% organic polymers , based on the total weight of the cellulosic biomass, in the cellulosic biomass into small molecular organic compounds. The synchronous hydrolyzation is catalytic hydrolyzation for which the catalyst used is a substance represented by L-M-S - H or L-M = S, wherein "M" represents metal, carbon or silicon, "S" represents a heteroatom, "L" represents one or more ligands, and "H" represents hydrogen.

## Description

### Technical Field

The present invention relates to a totally new method of refining cellulosic biomass, more particularly to a method capable of hydrolyzing synchronously all of the organic polymers in cellulosic biomass into small molecular organic compounds.

### Background Art

Global warming and depletion of fossil energy sources are tremendous challenges for human existence and development at present. Particularly, non-renewable energy sources such as fossil energy sources, for example, coal, oil, natural gas, are consumed at a rapidly increasing speed by human beings after this new century began. Currently, the organic carbon raw materials used in chemical engineering industry - the most important industry for human life - are substantially all originated from fossil energy sources. Cellulosic biomass is an annually renewable organic carbon source. It is of great significance for human beings to invent a technology for refining cellulosic biomass at a high conversion for preparation of aromatic compounds and liquid fuels as well as other chemical engineering raw materials.

### A Key Technology for Sustaining Human Existence and Development

Depletion of fossil energy sources (oil and coal) will occur for certain before human beings. The way to ensure the supply of organic carbon raw materials to chemical engineering industry on which human existence relies presently will have fatal influence on human existence and development after fossil energy sources are depleted. Those who first master the production technology and process in chemical engineering industry using cellulosic biomass as raw material will not only have an advantage in future worldwide competition, but also promote the cultural influence of their country for their find of the key technology for supporting the continuing existence and development of human beings.

### A Key Technology for Solving the Problem of Global Warming

The products of "cellulosic biomass refining" are intended to be used in such fields as chemical engineering, food additives, pesticides, fertilizers and the like, further in construction, vehicles, clothes, furniture, electronics, medical instruments, etc.. The direct effect of these uses in construction, vehicles, clothes, furniture, electronics, medical instruments, etc., is that carbon dioxide from the atmosphere does not return to the atmosphere any longer, equivalent to recovery of carbon dioxide from the atmosphere. A wide-range of promotion and application of the technology will relieve human beings from worrying about the use of fossil energy sources for there is enough carbon dioxide in the atmosphere to help the growth of green plants owing to the use of fossil energy sources. The recovery of carbon dioxide through the "cellulosic biomass refining" technology is far better than storage of compressed carbon dioxide underground, because no one can predict the leakage of carbon dioxide caused by earthquake, etc.. No scientific assessment on the risk of a sudden increase of carbon dioxide concentration in the atmosphere is yet available.

### A Key Technology for Solving the Three Rural Issues Concerning Agriculture, Countryside and Farmers

The critical points of the Three Rural Issues are employment and income.
The industrial development of "cellulosic biomass refining" will realize the conversion of crop straws/stalks into products of high additional value, and thus increase the income of the rural people.
In most cases, grass, reed and the like can grow in places unsuitable for growing corns. Therefore, plantable area can be increased on a large scale. In addition, the difficulty and the intensity of the field management during growth of most grasses are far less than those for corns, oil and sugar plants. Thus, there may be realized less input and more output, and the income of rural people can be increased further. Meanwhile, the labor intensity of the farmers will be decreased.

### A Key Technology for Solving the Problems of Environmental Pollution and Energy Shortage

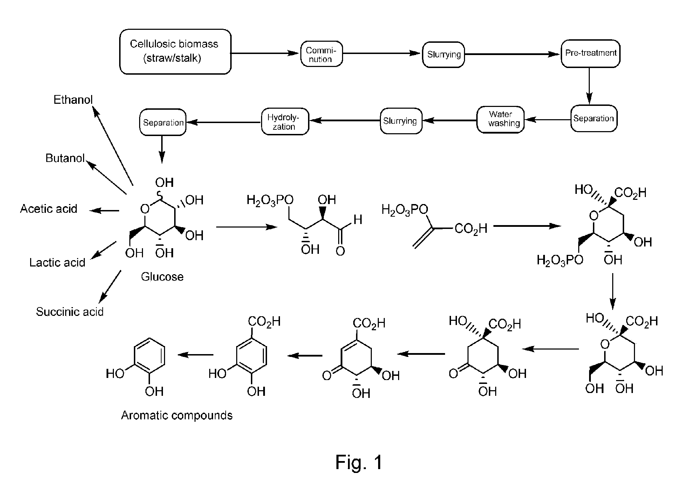

Unlike petrochemical engineering industry or coal chemical engineering industry, an all-around use of raw material has to be achieved by the chemical production technology of cellulosic biomass refining in order to ensure that profit can be made.
The practice of "squeezing it dry and eating it up", as a Chinese folksay puts it, ensures zero pollution of the cellulosic biomass chemical industry per se. The chemical liquid fuel products from cellulosic biomass refining contain no sulfur (S) or nitrogen (N). The components containing sulfur (S) and nitrogen (N) in the liquid fuels derived from fossil and coal are exclusively aromatic compounds which can not be removed completely and are burned to produce sulfur oxides and nitrogen oxides as the main environmental pollutants.
As illustrated in Fig. 1, the presently known technologies for hydrolyzing cellulosic biomass all involve hydrolyzation step by step. Furthermore, there are still no methods or technologies for hydrolyzing lignin. The separation and pre-treatment of individual components consumes a lot of energy, produces a large quantity of wastes, and results in excessive loss of organic carbon.
Therefore, there is an urgent need in the art for a technology that can be used to hydrolyze all of the organic polymer components in cellulosic biomass into small molecular organic compounds synchronously without the need of pre-treatment steps, without the need of separation of various kinds of organic polymers in cellulosic biomass in multiple steps, and without the loss of organic carbon during hydrolyzation.

### Summary

The first object of the invention is to provide a technology that can be used to hydrolyze all of the organic polymer components in cellulosic biomass into small molecular organic compounds synchronously without the need of pre-treatment steps, without the need of separation of various kinds of organic polymers in cellulosic biomass in multiple steps, and without the loss of organic carbon during hydrolyzation.

The second object of the invention is to provide a use of a catalyst which can be used to hydrolyze all of the organic polymer components in cellulosic biomass into small molecular organic compounds synchronously without the loss of organic carbon during hydrolyzation.

The invention provides a method of refining cellulosic biomass, wherein, based on the total weight of the cellulosic biomass, at least 85wt% organic polymers in the cellulosic biomass are hydrolyzed synchronously into small molecular organic compounds.
In a preferred embodiment, based on the total weight of the cellulosic biomass, at least 90wt% organic polymers in the cellulosic biomass are hydrolyzed synchronously into small molecular organic compounds.
In a preferred embodiment, based on the total weight of the cellulosic biomass, at least 99wt% organic polymers in the cellulosic biomass are hydrolyzed synchronously into small molecular organic compounds.

In a specific embodiment of the invention, the synchronous hydrolyzation is catalytic hydrolyzation in which a catalyst selected from the substances shown as Formula 1 or 2 is used, 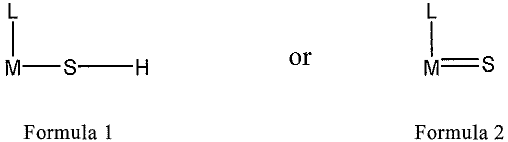 wherein
"M" represents metal, carbon or silicon;
"S" represents a heteroatom;
"L" represents one or more ligands; and
"H" represents hydrogen.

In a specific embodiment of the invention, the heteroatom is selected from "oxygen O", "sulfur S" or "nitrogen N".

In a specific embodiment of the invention, the catalyst may also be a water-soluble substance that can be converted into a structure of "Formula 1" or "Formula 2" when it is incorporated into water phase.

In a specific embodiment of the invention, the substance that can be converted into a structure of "Formula 1" or "Formula 2" is a metal inorganic salt, a metal organic salt or a combination thereof.

In a specific embodiment of the invention, the cellulosic biomass is any biomass containing cellulose, including but not limited to
Woody plants;
Herbaceous plants;
Cellulose-containing offal from agriculture, forestry, vegetable and fruit processing industries;
Dejecta of various animals;
Cellulose-containing offal from traditional Chinese medicine processing industry;
Crop castoffs; or
Combinations thereof.

In a preferred embodiment, the cellulosic biomass is selected from
Hard wood, soft wood, barks, leaves, roots, canes, wild grass, reed, bamboo, water plants; or
Cellulose-containing offal from agriculture, forestry, vegetable and fruit processing industries, dejecta of various animals and cellulose-containing offal from traditional Chinese medicine processing industry;
Crop castoffs including but not limited to corn stalk, sorghum stalk, wheat straw, bean stalk, rape stalk, peanut seedling, yam seedling, herbaceous fruit seedling and cotton stalk; or
Mixtures of the above biomass.

In a preferred embodiment, the main organic polymers contained in the cellulosic biomass include cellulose, lignin, hemicellulose, protein and the like.

In a specific embodiment of the invention, the synchronous hydrolyzation is carried out in the presence of an alkaline substance.
In a preferred embodiment, the alkaline substance is an inorganic base such as aqueous ammonia, a metal hydroxide, an alkali metal oxide, a metal carbonate, or various organic bases of ammonia.

In a specific embodiment of the invention, the small molecular organic compounds are monosaccharides and polysaccharides, organic carboxylic acids, phenols and substituted phenols, amino acids, cyclopentanone and cyclopentanol.

In another aspect, the invention provides a use of a catalyst which is used to hydrolyze all of the organic polymers in cellulosic biomass into small molecular organic compounds synchronously;
wherein the catalyst is selected from the substances shown as Formula 1 or 2, 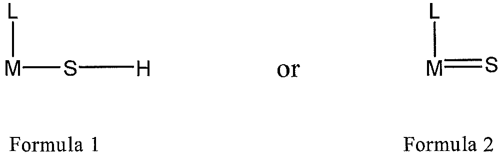 wherein
"M" represents metal, carbon or silicon;
"S" represents a heteroatom;
"L" represents one or more ligands; and
"H" represents hydrogen.

### Brief Description of the Drawings

Fig. 1 is a flowchart of a cellulosic biomass refining process known in prior art, characterized by multiple steps, necessity of pre-treatment and separation of components, unvaried products, and high cost.
Fig. 2 is a liquid chromatogram of the organic acid products obtained via the method of hydrolyzing synchronously the organic polymers in cellulosic biomass (wheat straw) according to the invention.
   Instrument type: Agilent HPLC 1200
   Chromatographic column: SB-AQ Reversed Phase Column
   Detector: DAD detector Detection wavelength: 210nm
   Mobile phase: aqueous solution of sodium dihydrogen phosphate 0.025M Flow rate: 0.6ml/min Injection volume : 5µL
Fig. 3 is a GCMS chromatogram of the aromatic compounds obtained via the method of hydrolyzing synchronously the organic polymers in cellulosic biomass (wheat straw) according to the invention.
   Instrument type: Agilent GC 7890A
   Detector: Agilent MSD 5975C
   Chromatographic column: DB-5
   Ramp program: Initial: 60°C Ramp 1 : 2°C/min to: 200°C Hold: 5min
   Ramp 2: 30°C/min to: 280°C Hold: 6min.

### Best Modes for Carrying Out the Invention

After extensive and intensive study, the inventors have invented a totally new catalysis system and obtained a technology that can be used to hydrolyze all of the organic polymer components in cellulosic biomass into small molecular organic compounds synchronously without the need of pre-treatment steps, without the need of separation of various kinds of organic polymers in cellulosic biomass in multiple steps, and without the loss of organic carbon during hydrolyzation. The invention is completed on this foundation.

Various aspects of the invention will be described in detail as follows.
Unless otherwise specified, all of the raw materials used in the invention are commercially available, or may be prepared according to conventional methods in the art. Unless otherwise defined or specified, all special and scientific terms used herein have the same meaning as that familiar to those skilled in the art. In addition, any method and material similar or equivalent to those cited herein may be used in the method of the invention.

### Synchronous hydrolyzation

The invention provides a method of refining cellulosic biomass, wherein, based on the total weight of the cellulosic biomass, at least 85wt% organic polymers in the cellulosic biomass are hydrolyzed synchronously into small molecular organic compounds.
In a preferred embodiment, based on the total weight of the cellulosic biomass, at least 90wt% organic polymers in the cellulosic biomass are hydrolyzed synchronously into small molecular organic compounds.
In a preferred embodiment, based on the total weight of the cellulosic biomass, at least 99wt% organic polymers in the cellulosic biomass are hydrolyzed synchronously into small molecular organic compounds.
Most preferably, based on the total weight of the cellulosic biomass, all of the organic polymers in the cellulosic biomass are hydrolyzed synchronously into small molecular organic compounds.
According to the invention, the "organic polymers" (i.e. organic polymer components) include cellulose, lignin, hemicellulose, protein or combinations thereof.
According to the invention, those skilled in the art can understand that the "small molecular organic compound" is a well-known term in the art, the variety and yield of which, as a hydrolytic product, depends on the particular components of the cellulosic biomass. The "small molecular organic compounds" include but are not limited to monosaccharides and polysaccharides, organic carboxylic acids, phenols and substituted phenols, amino acids, cyclopentanone and cyclopentanol. The "monosaccharides and polysaccharides, organic carboxylic acids, phenols and substituted phenols, amino acids, cyclopentanone and cyclopentanol" are hydrolytic products well known to those skilled in the art. The substituted phenols include but are not limited to o-methoxyl phenol, 2,6-dimethoxyl phenol, catechol, p-ethyl catechol, etc..

A totally new technical conception is adopted in the invention, wherein the steps of pre-treating cellulosic biomass are not carried out, the multiple-step separation of the various kinds of organic polymers in cellulosic biomass is exempted, while all of the organic polymer components in cellulosic biomass are hydrolyzed synchronously into small molecular organic compounds without the loss of organic carbon during hydrolyzation.

### Cellulosic biomass

The cellulosic biomass according to the invention is well known in the art. Specifically, the cellulosic biomass is defined as biomass containing cellulose, the main organic polymer components of which include cellulose, lignin, hemicellulose, protein and the like. In general, the common cellulosic biomass include various woody and herbaceous plants, such as hard wood, soft wood, barks, leaves, roots, canes, wild grass, reed, bamboo, water plants; cellulose-containing offal from agriculture, forestry, vegetable and fruit processing industries, cellulose-rich dejecta of various animals, and cellulose-containing offal from traditional Chinese medicine processing industry; crop castoffs such as corn stalk, sorghum stalk, wheat straw, bean stalk, rape stalk, peanut seedling, yam seedling, herbaceous fruit seedling and cotton stalk; etc..
More importantly, there are no methods or technologies for hydrolyzing lignin so far. However, the synchronous hydrolyzation method of the invention is directed to all of the polymers, including lignin, in cellulosic biomass.

### Catalyst

In an embodiment of the invention, the catalyst has a structure as follows: 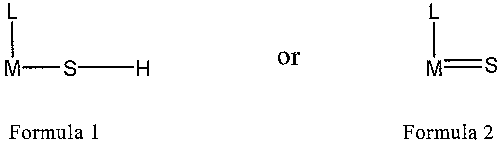 wherein
"M" represents metal, carbon or silicon;
"S" represents a heteroatom such as "oxygen O", "sulfur S", "nitrogen N", etc.;
"L" represents one or more ligands; and
"H" represents hydrogen.

The inventors of the invention provide a totally new catalysis system, wherein a catalyst in its reduced state is shown by Formula 1, while the oxidized state is shown by Formula 2, which states can be transformed from one to the other. Unlike a traditional catalyst which remains in the same oxidation state in the process of catalysis, the catalyst in the catalysis system of the invention is required to be in reduced state in order to hydrolyze one type of bonds and is oxidized after hydrolyzation, while it is needed at the same time to be in oxidized state so as to catalytically hydrolyze another type of bonds and is reduced after hydrolyzation, for the bonds to be catalytically hydrolyzed include acetal bonds, ether bonds, etc.. These two states promote each other, and such a catalysis is called mutually promoting catalysis. This is the first time in the world to find such a catalysis system. That's to say, it can be construed that the substance added into the reaction in the state of Formula 1 may be transformed into the state of Formula 2. Conversely, the state of Formula 2 may be transformed into that of Formula 1.
The metal is not particularly limited on condition that it does not impact the conversion of acetal bonds and ether bonds in the invention. For example, it includes copper, palladium, platinum, nickel, mercury and the family members thereof.
The ligand may be one or more. The ligand is not particularly limited on condition that it does not impact the conversion of acetal bonds and ether bonds in the invention.

Since the compounds of Formulae 1 and 2 can be transformed mutually into each other, it can be construed that the catalyst may be:
A compound having the structure of Formula 1 per se;
A compound having the structure of Formula 2 per se;
Also a water-soluble substance that can be transformed into the structure of "Formula 1" after it is incorporated into water phase; and
Also a water-soluble substance that can be transformed into the structure of "Formula 2" after it is incorporated into water phase.

More particularly, for example, the catalyst is a metal chloride, specifically, such as copper chloride, palladium chloride, platinum chloride, nickel chloride, etc.. It may also be a metal organic acid salt, such as mercury acetate, copper acetate, etc.. The organic acid salt may also be selected from other water-soluble organic acid salts. But it must be water-soluble to the extent that it can be dissolved in water and transformed into L-M-OH or L-M=O. One that is not water-soluble or can not be transformed into the substance of Formula 1 or 2 is not the catalyst.

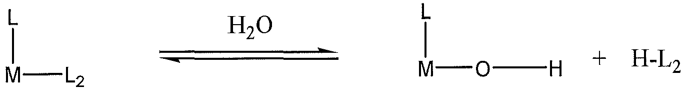

The various types of catalysis systems used in the invention are exemplified as follows.
L-M=S catalysts. An example is sodium perrhenate, wherein its stable formula in common state is L-Re=O , and all of the other elements are ligands.
L-M-SH catalysts.For example, this type of catalysts may be prepared using ligands and metal salt. For example, M=Mn , L is large ligand , and -OH is derived from water, the solvent.
L-M-SH catalysts formed in water. For example, mercury trifluoroacetate is not a substance of Formula 1 or 2, but can be transformed into a catalyst of L-M-OH quickly in water, wherein M=Hg, and OH is derived from water.
Organic compounds, such as quinone compounds, may be used, for example, 2,3-dichloro-5,6-dicyanobenzoquinone (DDQ), the stable structure of which per se in common state is L-M=S, wherein M=C; for another example, anthraquinone, the stable structure of which in common state is L-M-SH, wherein M=C.

The amount of the catalyst used is generally 0.1%-100 (by weight of the cellulosic biomass, based on the molecular weight of the compound of Formula 1 or Formula 2). Although the reaction may also be catalyzed if the amount of the catalyst used is out of the above range, the amount of the catalyst used that is lower than this range is not preferred due to slow reaction rate, and the amount of the catalyst used that is higher than this range is neither preferred due to excessively high cost.
More preferably, the amount of the catalyst used is generally 0.1%-5%.

### Alkaline Substance

Due to the generation of organic acid during hydrolyzation reaction, certain amount of base is usually added in the hydrolyzation reaction to neutralize the organic acid generated in the reaction so as to ensure the normal running of the reaction. Any base can be used, including inorganic and organic ones. Inorganic compounds include aqueous ammonia, metal hydroxides, alkali metal oxides, metal carbonates, etc.; and organic compounds include various ammonia (amines?).
The amount of the alkaline substance used is not particularly limited on condition that it does not impact the conversion of acetal bonds and ether bonds by the catalyst of the invention.
Particularly, the amount includes but is not limited to 10% - 200%, preferably 20 - 90% , based on the amount of the cellulosic biomass used.

### Reaction Conditions

In an embodiment of the invention, the reaction temperature for hydrolyzing cellulosic biomass simultaneously is generally higher than 100°C but lower than 280°C. A temperature lower than 100°C is not preferred, for the hydrolyzation reaction is too slow. A temperature higher than 280°C is neither preferred, for a portion of the organic carbon will undergo carbonization and gasification reactions. Preferably, the temperature is 160-270.
The pressure for the hydrolyzation reaction is generally in the range from ambient pressure to 10MPa. The principle for selection of the pressure is such that the hydrolyzation reaction system is in liquid state during the hydrolyzation reaction. Too high a pressure will add to the cost of the hydrolyzation reaction. Preferably, the pressure is 1-7 MPa.

The above reaction conditions are only part of the reaction conditions of the invention. According to the above examples, those skilled in the art may realize synchronous hydrolyzation by adjusting other parameters.
Owing to the disclosure herein, other aspects of the invention will be obvious to those skilled in the art.
The invention will be further illustrated with reference to the following specific examples. It is to be understood that these examples are merely intended to demonstrate the invention without limiting the scope of the invention. The experimental methods in the following examples for which no specific conditions are indicated will be carried out generally according to national standards. If no national standard is available, they will be carried out according to general international standards, under conventional conditions or under those conditions suggested by the manufacturers. Unless otherwise specified, all parts and percentages are based on weight, and the molecular weight of the polymers is number average molecular weight.

The examples given below serve to demonstrate the invention better, and do not suggest that the disclosure of the invention is limited to the following examples. The catalysts disclosed herein include all substances in conformity to the structures described above.

### Example 1 : Hydrolyzation of Wheat Straw (Catalyst: sodium perrhenate NaReO₄) L-M=O catalyst

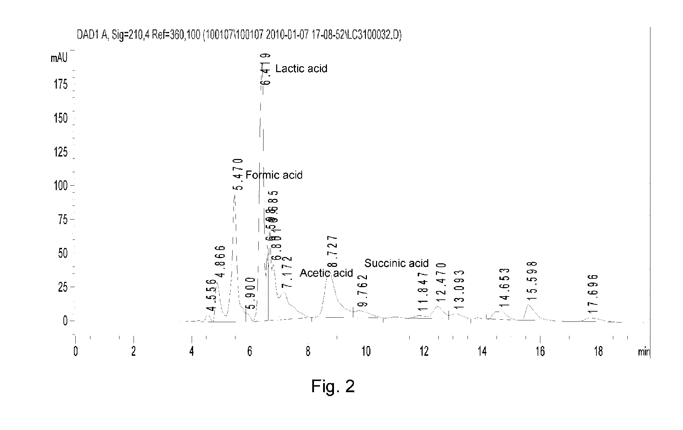

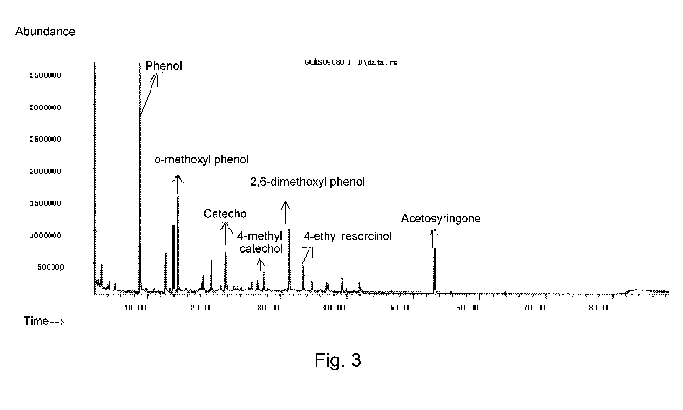

Into a 1000mL stainless steel autoclave were added 100g solid of comminuted dry wheat straw (about 2mm in particle size, containing 42% cellulose, 21% lignin and 20% hemicellulose), 1g sodium perrhenate and 900mL 6% aqueous solution of sodium hydroxide. The reaction system was sealed, heated to 260°C for 3 hours and then cooled to room temperature. There was no obvious change of the pressure before and after the reaction. After removal of the reaction product mixture, the reactor was washed twice with a small amount of pure water. No sign of carbonization reaction was observed in the reactor. The reaction mixture and the washing liquid were combined and filtered. The filter cake was washed twice with a small amount of pure water and vacuum dried. After analysis, the filter cake was found to be 7g in weight (containing 17% ash), and the unhydrolysis rate was 7%. The aqueous solution obtained after filtration was adjusted to pH=2.5-3.5 with hydrochloric acid, and passed through 140g non-ionic macroporous adsorption resin DA201C column (3cm in inner diameter). The adsorption column was washed with 200mL pure water. The aqueous solutions were combined and then dewatered. The resulting solid was extracted with dry ethanol to obtain 56g organic acid mixture (yield 56%). The product mainly comprised lactic acid, hydroxyl butyric acid, succinic acid, formic acid, acetic acid, etc., as shown in Fig. 2. After washed with water, the macroporous adsorption resin DA201C column was eluted with 500mL dry ethanol. After the ethanol in the resulting solution of aromatic compounds in ethanol was removed, the product weighed 16g (yield 16%) and mainly comprised phenol, o-methoxyl phenol, 2,6-dimethoxyl phenol, catechol, p-ethyl catechol, etc., as shown in Fig. 3. Based on the content of organic compounds in the cellulosic biomass, the overall yield was 86.7%.

### Example 2 : Hydrolyzation of Wheat Straw [Catalyst: mercury trifluoroacetate Hg(OCOCF₃)₂] transformable into L-M-OH catalyst in water

The operational procedure for the reaction was the same as in Example 1. Into a 1000mL stainless steel autoclave were added 100g solid of comminuted dry wheat straw (about 2mm in particle size, containing 42% cellulose, 21% lignin and 20% hemicellulose), 1g mercury trifluoroacetate and 900mL 6% aqueous solution of sodium hydroxide. The reaction system was sealed, heated to 260°C for 3 hours and then cooled to room temperature. There was no obvious change of the pressure before and after the reaction. After removal of the reaction product mixture, the reactor was washed twice with a small amount of pure water. No sign of carbonization reaction was observed in the reactor. The reaction mixture and the washing liquid were combined and filtered. The filter cake was washed twice with a small amount of pure water and vacuum dried. After analysis, the filter cake was found to be 3g in weight (containing 19% ash), and the unhydrolysis rate was 3%. The aqueous solution obtained after filtration was adjusted to pH=2.5-3.5 with hydrochloric acid, and passed through 140g non-ionic macroporous adsorption resin DA201C column (3cm in inner diameter). The adsorption column was washed with 200mL pure water. The aqueous solutions were combined and then dewatered. The resulting solid was extracted with dry ethanol to obtain 57g organic acid mixture (yield 57%). The product mainly comprised lactic acid, hydroxyl butyric acid, succinic acid, formic acid, acetic acid, etc.. After washed with water, the macroporous adsorption resin DA201C column was eluted with 500mL dry ethanol. After the ethanol in the resulting solution of aromatic compounds in ethanol was removed, the product weighed 18g (yield 18%) and mainly comprised phenol, o-methoxyl phenol, 2,6-dimethoxyl phenol, catechol, p-ethyl catechol, etc.. Based on the content of organic compounds in the cellulosic biomass, the overall yield was 90.3%.

### Example 3 : Hydrolyzation of Wheat Straw [Catalyst: L-Mn-OH] L-M-OH catalyst

### 1. Synthesis of the catalyst: The structure of the ligand L is shown below.

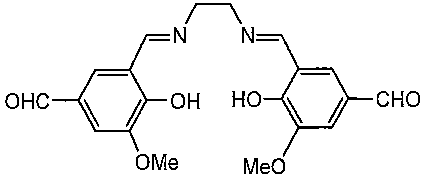

The solvent was 50% (v/v) solution of methanol in water. Into the solution were dissolved 4.9g manganese acetate tetrahydrate (2mmol) and 7.68g ligand (2mmol), and then added 30% aqueous solution of potassium hydroxide (4mmol, used to transform the phenolic hydroxyl group of the ligand into potassium salt). After reflux for 50min, a yellow brown solid product was filtered out. The product was washed several times with 50% solution of methanol in water and then vacuum dried. Element analysis: manganese (Mn) 12.18%, carbon (C) 52.63%, nitrogen (N) 6.38%. Formula: L-Mn-OH(MnC₂₀H₁₉N₂O₇) Theoretical contents: manganese (Mn) 12.09%, carbon (C) 52.87%, nitrogen (N) 6.17%. Infrared (IR, cm⁻¹)spectrum: 421(w,νMn-O), 878(m,γCH), 991(m,δCH), 1021(w,νC-O-C), 1085(m,δOH), 1152(s,νC-O), 1191(w,νC-O-C), 1273(vs,vC-N), 1391(m,δCH₃), 1451(m,δCH₂), 1600(vs,vC=C), 1624(vs, vC=N), 1679(m,vC=O).
The operational procedure for the synchronous hydrolyzation reaction was the same as in Example 1. Into a 1000mL stainless steel autoclave were added 100g solid of comminuted dry wheat straw (about 2mm in particle size, containing 42% cellulose, 21% lignin and 20% hemicellulose), 1g L-Mn-OH catalyst and 900mL 6% aqueous solution of sodium hydroxide. The reaction system was sealed, heated to 260°C for 3 hours and then cooled to room temperature. There was no obvious change of the pressure before and after the reaction. After removal of the reaction product mixture, the reactor was washed twice with a small amount of pure water. No sign of carbonization reaction was observed in the reactor. The reaction mixture and the washing liquid were combined and filtered. The filter cake was washed twice with a small amount of pure water and vacuum dried. After analysis, the filter cake was found to be 8g in weight (containing 15% ash), and the unhydrolysis rate was 8%. The aqueous solution obtained after filtration was adjusted to pH=2.5-3.5 with hydrochloric acid, and passed through 140g non-ionic macroporous adsorption resin DA201C column (3cm in inner diameter). The adsorption column was washed with 200mL pure water. The aqueous solutions were combined and then dewatered. The resulting solid was extracted with dry ethanol to obtain 48g organic acid mixture (yield 48%). The product mainly comprised lactic acid, hydroxyl butyric acid, succinic acid, formic acid, acetic acid, etc.. After washed with water, the macroporous adsorption resin DA201C column was eluted with 500mL dry ethanol. After the ethanol in the resulting solution of aromatic compounds in ethanol was removed, the product weighed 13g (yield 13%) and mainly comprised phenol, o-methoxyl phenol, 2,6-dimethoxyl phenol, catechol, p-ethyl catechol, etc.. Based on the content of organic compounds in the cellulosic biomass, the overall yield was 73.5%.

### Example 4 : Hydrolyzation of Wheat Straw [Catalyst: 2,3-dichloro-5,6-dicyanobenzoquinone, DDQ] L-M=O catalyst

The operational procedure of the reaction was the same as in Example 1.Into a 1000mL stainless steel autoclave were added 100g solid of comminuted dry wheat straw (about 2mm in particle size, containing 42% cellulose, 21% lignin and 20% hemicellulose), 1g DDQ and 900mL 6% aqueous solution of sodium hydroxide. The reaction system was sealed, heated to 260°C for 3 hours and then cooled to room temperature. There was no obvious change of the pressure before and after the reaction. After removal of the reaction product mixture, the reactor was washed twice with a small amount of pure water. No sign of carbonization reaction was observed in the reactor. The reaction mixture and the washing liquid were combined and filtered. The filter cake was washed twice with a small amount of pure water and vacuum dried. After analysis, the filter cake was found to be 3g in weight (containing 11% ash), and the unhydrolysis rate was 9%. The aqueous solution obtained after filtration was adjusted to pH=2.5-3.5 with hydrochloric acid, and passed through 140g non-ionic macroporous adsorption resin DA201C column (3cm in inner diameter). The adsorption column was washed with 200mL pure water. The aqueous solutions were combined and then dewatered. The resulting solid was extracted with dry ethanol to obtain 45g organic acid mixture (yield 45%). The product mainly comprised lactic acid, hydroxyl butyric acid, succinic acid, formic acid, acetic acid, etc.. After washed with water, the macroporous adsorption resin DA201C column was eluted with 500mL dry ethanol. After the ethanol in the resulting solution of aromatic compounds in ethanol was removed, the product weighed 13g (yield 13%) and mainly comprised phenol, o-methoxyl phenol, 2,6-dimethoxyl phenol, catechol, p-ethyl catechol, etc.. Based on the content of organic compounds in the cellulosic biomass, the overall yield was 69.8%.

### Example 5 : Hydrolyzation of Wheat Straw [Catalyst: anthraquinone] L-M-OH catalyst

The operational procedure of the reaction was the same as in Example 1.Into a 1000mL stainless steel autoclave were added 100g solid of comminuted dry wheat straw (about 2mm in particle size, containing 42% cellulose, 21% lignin and 20% hemicellulose), 1g anthraquinone and 900mL 6% aqueous solution of sodium hydroxide. The reaction system was sealed, heated to 260°C for 3 hours and then cooled to room temperature. There was no obvious change of the pressure before and after the reaction. After removal of the reaction product mixture, the reactor was washed twice with a small amount of pure water. No sign of carbonization reaction was observed in the reactor. The reaction mixture and the washing liquid were combined and filtered. The filter cake was washed twice with a small amount of pure water and vacuum dried. After analysis, the filter cake was found to be 7g in weight (containing 11% ash), and the unhydrolysis rate was 7%. The aqueous solution obtained after filtration was adjusted to pH=2.5-3.5 with hydrochloric acid, and passed through 140g non-ionic macroporous adsorption resin DA201C column (3cm in inner diameter). The adsorption column was washed with 200mL pure water. The aqueous solutions were combined and then dewatered. The resulting solid was extracted with dry ethanol to obtain 46g organic acid mixture (yield 46%). The product mainly comprised lactic acid, hydroxyl butyric acid, succinic acid, formic acid, acetic acid, etc.. After washed with water, the macroporous adsorption resin DA201C column was eluted with 500mL dry ethanol. After the ethanol in the resulting solution of aromatic compounds in ethanol was removed, the product weighed 14g (yield 14%) and mainly comprised phenol, o-methoxyl phenol, 2,6-dimethoxyl phenol, catechol, p-ethyl catechol, etc.. Based on the content of organic compounds in the cellulosic biomass, the overall yield was 72.2%.

### Example 6 : Synchronous catalytic hydrolyzation of different cellulosic biomass sources

The operational procedure of the reaction was the same as in Example 1.
The catalyst was palladium acetate [Pd(OAc)₂], and the results were shown in the table below.

| Cellulosic biomass | Unhydrolysis rate (%) | Ash content of unhydrolyzed solid (%) | Output of organic acids (g) | Output of aromatic compounds (g) | Overall yield (%) |
|---|---|---|---|---|---|
| Wheat stalk | 9 | 17 | 55 | 16 | 85.5 |
| Corn stalk | 7 | 22 | 59 | 13 | 86.7 |
| Bean stalk | 8.9 | 19 | 64 | 13 | 92.8 |
| Bagasse | 6 | 12 | 61 | 14 | 90.3 |
| Reed | 9 | 14 | 56 | 17 | 87.9 |
| Pennisetum | 8 | 19 | 59 | 12 | 85.5 |
| Moso bamboo | 5.6 | 11 | 52 | 22 | 89.1 |
| Pine wood | 7.1 | 11 | 48 | 23 | 85.5 |
| Poplar wood | 9 | 14 | 52 | 13 | 78.3 |
| Rape stalk | 8.3 | 19 | 49 | 13 | 74.6 |

The above description just puts forward some preferred embodiments of the invention, and is not intended to limit the scope of the essential technological matter of the invention. The scope of the essential technological matter of the invention is broadly defined by the claims of the application. Any technical entity or method fulfilled by others is to be considered as being included in the scope of the claims of the application if it is totally the same as or an equivalent variation of any of those defined in the claims.
All of the documents mentioned in the invention are incorporated herein by reference, as if each of them were incorporated herein individually by reference. It is further to be understood that various changes or modifications can be made by those skilled in the art after reading the above disclosure of the invention, and these equivalent variations fall in the scope defined by the accompanied claims of the application as well.

## Claims

1. A method of refining cellulosic biomass, wherein, based on the total weight of the cellulosic biomass, at least 85wt% organic polymers in the cellulosic biomass are hydrolyzed synchronously into small molecular organic compounds.

2. The method of Claim 1, wherein the synchronous hydrolyzation is catalytic hydrolyzation, wherein the catalyst used in the catalytic hydrolyzation is selected from the group consisting of substances shown as Formulae 1 and 2: 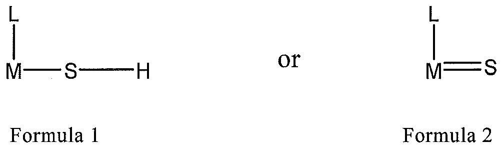 wherein
"M" represents metal, carbon or silicon;
"S" represents a heteroatom;
"L" represents one or more ligands; and
"H" represents hydrogen.

3. The method of Claim 2, wherein the heteroatom is selected from the group consisting of "oxygen O", "sulfur S" and "nitrogen N".

4. The method of Claim 2, wherein the catalyst is a water-soluble substance which can be converted into a structure of "Formula 1" or "Formula 2" when incorporated into water phase.

5. The method of Claim 4, wherein the substance which can be converted into a structure of "Formula 1" or "Formula 2" is a metal inorganic salt, a metal organic salt or a combination thereof.

6. The method of Claim 1, wherein the cellulosic biomass is any biomass containing cellulose,
preferably, the cellulosic biomass includes:
woody plant;
herbaceous plant;
cellulose-containing offal from agriculture, forestry, vegetable or fruit processing industries;
dejecta of various animals;
cellulose-containing offal from traditional Chinese medicine processing industry;
crop castoffs; or
combinations thereof.

7. The method of Claim 6, wherein the cellulosic biomass contains lignin.

8. The method of Claim 2, wherein the synchronous hydrolyzation is carried out in the presence of an alkaline substance.

9. The method of Claim 1, wherein the small molecular organic compounds are monosaccharides as well as polysaccharides, organic carboxylic acids, phenols as well as substituted phenols, amino acids, or cyclopentanone as well as cyclopentanol.

10. A use of a catalyst, wherein the catalyst is used to hydrolyze all of the organic polymers in cellulosic biomass into small molecular organic compounds synchronously;
wherein the catalyst is selected from the group consisting of substances shown as Formulae 1 and 2, 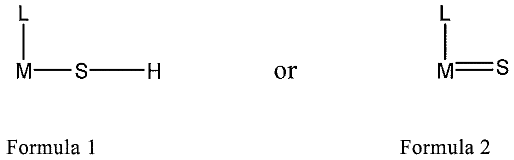 wherein
"M" represents metal, carbon or silicon;
"S" represents a hybrid atom;
"L" represents one or more ligands; and
"H" represents hydrogen.
